(19) Europäisches Patentamt
European Patent Office
Office européen des brevets

(11) **EP 0 179 075 B2**

(12) # NEW EUROPEAN PATENT SPECIFICATION

(45) Date of publication and mention
of the opposition decision:
**15.10.1997 Bulletin 1997/42**

(45) Mention of the grant of the patent:
**19.05.1993 Bulletin 1993/20**

(21) Application number: **85901234.6**

(22) Date of filing: **19.02.1985**

(51) Int Cl.⁶: **C07K 14/805**, A61K 38/42

(86) International application number:
**PCT/US85/00251**

(87) International publication number:
**WO 85/04407 (10.10.1985 Gazette 1985/22)**

(54) **VIRUS RISK-REDUCED HEMOGLOBIN AND METHOD FOR MAKING SAME**

HÄMOGLOBIN MIT REDUZIERTEM VIRUSRISIKO UND DESSEN HERSTELLUNG

HEMOGLOBINE A RISQUE REDUIT DE CONTAMINATION VIRALE ET SON PROCEDE DE PREPARATION

(84) Designated Contracting States:
**AT BE CH DE FR GB LI LU NL SE**

(30) Priority: **23.03.1984 US 592633**

(43) Date of publication of application:
**30.04.1986 Bulletin 1986/18**

(73) Proprietor: **BAXTER INTERNATIONAL INC.**
**(a Delaware corporation)**
**Deerfield Illinois 60015 (US)**

(72) Inventor: **ESTEP, Timothy, N.**
**Lindenhurst, IL 60046 (US)**

(74) Representative: **MacGregor, Gordon et al**
**ERIC POTTER CLARKSON**
**St. Mary's Court**
**St. Mary's Gate**
**Nottingham, NG1 1LE (GB)**

(56) References cited:
| EP-A- 71 888 | EP-A- 0 035 204 |
| EP-A- 0 123 877 | US-A- 3 864 478 |
| US-A- 4 001 401 | US-A- 4 291 020 |
| US-A- 4 370 264 | US-A- 4 377 512 |
| US-A- 4 438 098 | US-A- 4 439 357 |
| US-A- 4 456 590 | |

• **N, J Lab. Clin. Med., v 33, issued 1948, Daland et al., see pages 1085 and 1086**

**EP 0 179 075 B2**

**Description**

This invention relates to methods for inactivating viruses in hemoglobin preparations. In particular it is concerned with destroying the biological infectivity of such viruses while minimally inactivating the biological activity of hemoglobin.

Various literature and patent sources contain disclosures of methods for inactivating viruses in blood plasma protein fractions. An effective method employs dry heat inactivation, i.e., the lyophilized protein which is suspected to bear viral contamination is simply heated in the dry state at temperatures above about 50°C until the desired viral inactivation is achieved. A representative method of this sort is disclosed in PCT publication WO 82/03871.

Another technique also employs heat, but the protein fraction is heated while in aqueous solution rather than dry. Stabilizers are included in the solutions in order to preserve the biological activity of the desired protein. For example, see U.S. patents 4,297,344 and 4,327,086 and European patent applications 53,338 and 52,827. The stabilizers that have been used for this purpose are glycine, alpha- or beta- alanine, hydroxyproline, glutamine, alpha-, beta- or gamma-aminobutyric acid, monosaccharides, oligosaccharides, sugar alcohols, organic carboxylic acids, neutral amino acids, chelating agents and calcium ions. No known publication discloses or suggests the use of antioxidants as stabilizers in viral thermal inactivation processes.

These methods are both founded on the discovery that heat will inactivate viruses at a greater rate than the labile proteins studied provided, however, in the case of heating in solution that an agent or stabilizer is present which stabilizes the desired protein but which does not at the same time similarly stabilize the viral contaminants.

Antioxidants have been used in association with hemoglobins in the past. Hollocher, "J. Biol. Chem." 241:9 (1966) observe that thiocyanate decreases the heat stability of hemoglobin.

European Patent Application 78961 teaches stabilizing crosslinked hemoglobin against oxidation by the use of an antioxidant.

Daland et al., "J. Lab. Clin. Med." 33:1082-1088 (1948) employs a reducing agent to reduce red blood cell hemoglobin in order to assay for sickle cell anemia.

Sodium ascorbate was disclosed to be ineffective in protecting the hemoglobin molecule from deterioration during prolonged storage. Rabiner et al., "Ann. Surg." 171:615 (1970).

Hemoglobin solutions have been proposed for use as blood substitutes, either as a solution of crystalline hemoglobin or as a polymer crosslinked to other hemoglobin or other macromolecules such as polysaccharides. See for example U.S. patents 4,001,401; 4,061,736; 4,053,590; and 4,001,200; and West German offenlegungsschiften 3029307 and 2616086. All of these products are obtained by processes which use human red blood cells from whole blood as a starting material. The hemoglobin is separated from the formed matter (including stroma) of the red cells by lysis and centrifugation, followed by processing in accord with known techniques, including substitution with pyridoxal groups. These methods are not concerned with assuring that any viruses present in the whole blood starting material do not carry forward into the final hemoglobin product in infectious form. It is well known that whole blood can contain hepatitis A, B and non - A, non - B virus, cytomegalovirus, Epstein - Barr virus and, it is speculated, a viral etiological agent for acquired immunedeficiency syndrome (AIDS).

It is an object of this invention to reduce the risks of infection of patients by adventitious viral contaminants in hemoglobin - containing composition administered to such patients. It is a further object to do so without denaturing a substantial proportion of the hemoglobin so that it becomes incapable of performing its oxygen transport function in vivo.

The term hemoglobin as used below is generic for oxy-, carboxy- and deoxyhemoglobin, as well as pyridoxalated (covalently bonded to pyridoxal groups by reaction with pyridoxal-5'-phosphate) and/or crosslinked derivatives thereof unless otherwise stated. Crosslinked derivatives include glutaraldehyde, ring-opened diol, or 3,5-dibromosalicyl-bis-fumarate (DBBF) crosslinked hemoglobin. These hemoglobin derivatives are well - known.

Summary

I have now discovered a method for reducing the risk of biologically infectious virus in hemoglobin-containing compositions, which comprises heating (a) the composition and (b) a reducing agent under conditions such that the virus is rendered substantially biologically inactive without substantially biologically inactivating the hemoglobin. The product of this process contains biologically active hemoglobin, heat inactivated virus residues and is substantially free of biologically inactive hemoglobin. The final products also may contain the reducing agent. And the composition may be dry or aqueous solutions.

Brief Description of the Drawing

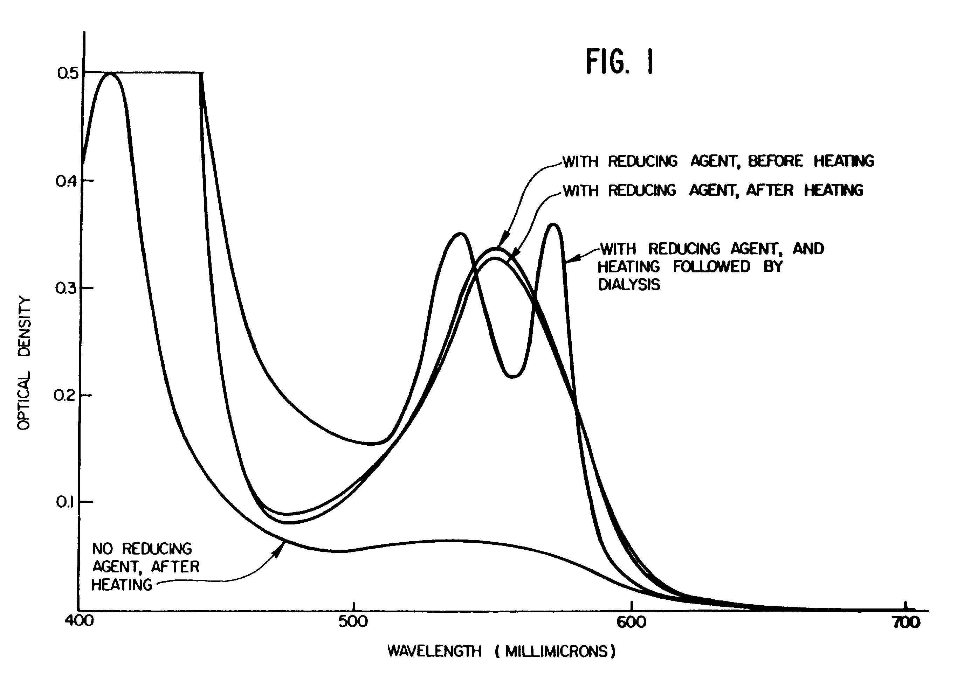

Fig. 1 discloses the stabilizing effect of dithionite reducing agent on hemoglobin after heating in solution at 56°C for 10 hours.

2

Detailed Description

Biologically active hemoglobin is hemoglobin which is capable of performing in vivo the oxygen transport function of native hemoglobin. However, it is not necessary for the hemoglobin to function with the efficacy found in its red blood cell environment. Rather, a comparison is made between the material without the heat treatment herein and a comparable lot after such heat treatment. This comparison can be made with in vivo or in vitro assays already known in the art, for example measurement of arteriovenous oxygen differences in the rat after exchange transfusion with the test composition or by changes in the adsorption spectrum of the hemoglobin before and after treatment. Hemoglobin that is biologically inactive, for example, may have been converted to methemoglobin, had its protein component denatured or otherwise adversely impacted by heat.

Hemoglobin compositions include the hemoglobin derivatives discussed above as well as native, substantially purified hemoglobin, including crystalline hemoglobin. Ordinarily one will not be interested in methemoglobin or its derivatives because they are not biologically active as defined in the preceding paragraph. Suitable hemoglobin compositions ideally will contain at least 99% hemoglobin by weight of total protein, total phospholipid content of less than about 3 ug/ml, less than about 1 ug/ml of either phosphatidylserine or phosphatidylethanolamine, inactive heme pigment of less than 6%, an oxygen affinity ($P_{50}$) of about from 24 to 32 mm Hg (37°C, pH 7.4, $pCO_2$ of 40 mm Hg, and 1 mM hemoglobin) with a Hill's constant of at least about 1.8 and an oxygen combining capacity of at least about 17 ml $O_2$/dl of hemoglobin solution. These specifications are not critical; others may be employed.

The hemoglobin composition generally will be dissolved in water at a concentration of about from 1 to 40 g/dl, preferably about from 1 to 14.5 g/dl prior to heat treatment. The concentration selected will depend upon whether the solution is intended to be used as such for therapeutic use or to be further processed by ultrafiltration and the like, or lyophilized. In the later situations the concentration can he any that is conveniently handled in the subsequent processing steps. Where the product is to be infused it preferably will have concentration of about from 13.5 to 14.5 g hemoglobin composition/dl.

The reducing agent is a substance or chemical or physical intervention that maintains the hemoglobin in the deoxy form during heating, but generally is a chemireductant which should be physiologically acceptable and will have a reducing potential greater than or more effective than ascorbate. I have found that ascorbate is relatively ineffective in heat stabilizing hemoglobin for the purposes herein. Reduced redox dyes and sulfhydryl or sulfoxy compounds include many acceptable agents. Suitable reducing agents are alkali metal dithionite, bisulfite, metabisulfite or sulfite, reduced glutathione and dithiothreitol. Dithionite is preferred. The agents easily may be assayed for efficacy by the method shown in Example 2 below.

The quantity of reducing agent to be included in the aqueous solution of the hemoglobin composition will vary depending upon the reducing strength of the agent, the quantity of hemoglobin, the estimated viral burden (and as a consequence the intensity of the heat treatment), the presence of oxidizing solutes and oxygen, and other factors as will be apparent to the skilled artisan. Accordingly, the optimal concentration will be determined by routine experiments. This can be done by following the in vitro changes in hemoglobin spectrum as described below in Example 2 so that only sufficient reducing agent is included to preserve a substantial proportion of the biological activity of the hemoglobin under the viral inactivation conditions, but no more than that amount. Dithionite may be used in a concentration of about from 10 to 100 mM in the hemoglobin solutions, preferably about from 20 to 40 mM.

Various additives may be present in the composition in addition to the reducing agent. For example, buffers such as phosphate, bicarbonate or tris (to pH of about from 7-8), inorganic ions in concentrations generally less than found in plasma (e.g., sodium and chloride at concentrations of no more than about 150 meq/l each) and lyophilization stabilizers such as amino acids or saccharides. One should use nonreducing sugars such as mannose or sugar alcohols when lyophilized hemoglobin compositions are heat treated.

The heat treatment of the hemoglobin solution containing the reducing agent may be effected by any method for heating such as microwave or infrared radiation, or thermal contact by such devices as resistance heaters or water baths. The temperature of the composition is increased as rapidly as possible consistent with relatively uniform heating, in order to avoid localized overheating, up to a viral inactivating temperature. This temperature will range about from 50°C to 80°C, preferably about 60°C, if the inactivation is to occur over a reasonably brief period of time. This typically will range about from 5 to 15 hours, preferably 10 hours for solutions and about from 20 to 96 hours for dry compositions. The shorter incubations will be used with higher temperatures. The time and temperature of inactivation will depend upon a number of factors such as the viral bioburden, the protein concentration, the nature of the hemoglobin (cross-linked or not) and the reducing agent concentration. The efficacy of the treatment process for viral kill is best assayed by seeding an aliquot of the composition to be treated with a candidate virus noninfective for man such as animal viruses or bacteriophage, for example sindbis, cytomegalovirus or T4. Suitable methods for such assays are disclosed in PCT publication WO 82/03871. The reducing agent concentration and the time and temperature of candidate virus inactivation are balanced against the loss of hemoglobin biological activity to arrive at the optimal conditions for heat inactivation. The inactivation of candidate virus should proceed until a reduction of at least 3 and preferably 6 logs of viral activity has been achieved. This can be accomplished by the method herein without a substantial loss in hemoglobin biological activity, i.e. only about from 1 to 15 mole percent of hemoglobin is rendered biologically inactive in the ordinary case.

The resulting product will contain biologically active hemoglobin, will be substantially free of biologically inactive hemoglobin and will contain biologically noninfectious virus. The residues of biologically noninfections virus may be detected by immune assays for viral antigens, which are generally not immunologically destroyed by the process, as compared to in vivo (tissue culture or host animal) viral infectivity assays. The presence of viral antigens coupled with a loss in or substantial lack of viral infectivity is an indicia of products treated in accord with this process.

Heat treated solutions are processed in order to make them convenient for therapeutic use. Dilute hemoglobin solutions may be concentrated by ultrafiltration and/or lyophilization. Ultrafiltration is useful also if necessary to remove excess reducing agent, i.e. to reduce the concentration of reducing agent to a physiologically acceptable level. This will ordinarily be on the order of less than about 5 mM, but the exact amount will depend on the estimated rate of infusion and the character of the reducing agent. For example, reduced glutathione is relatively innocuous and may remain in the composition in relatively high proportions.

The heat treatment step can be performed before or after the pyridoxalation or cross - linking referred to above. Preferably the hemoglobin is pyridoxalated and crosslinked before heat treatment. This helps to ensure that any viral contamination which may occur during manufacturing is also dealt with. If the amount of reducing agent used during heat treatment is physiologically acceptable then the heating can occur in final filed containers such as bags or vials.

The hemoglobin composition is advantageously in aqueous solution when heat treated but dry composition also can be heat treated. For example, if the hemoglobin composition is intended for long-term storage it may be lyophilized or dried from a solution containing the reducing agent, and then heated.

The following examples are intended to be illustrative and should not be construed as limiting the scope of the invention.

EXAMPLE 1

This contemplated procedure is illustrative of the manner in which hemoglobin compositions may be treated in accord with this invention. A solution is prepared which contains 1g% stroma-free hemoglobin, 30 mM of sodium dithionite ($Na_2 S_2 O_4$)and sufficient sodium - bicarbonate buffer (.1 to .3M) to maintain the pH at 7.5. 100 ml of this solution is sealed in a glass vial so as to leave no gas head space and then heated at 60°C for 10 hours by immersion in a water bath. After heating, the solution is removed from the vial, diafiltered over a 30,000 MW cutoff membrane to remove excess dithionite and to adjust the ionic content of the medium, concentrated by ultrafiltration to a hemoglobin content of 14 g/dl, and passed through a 0.2 micron filter to remove any particulate matter and to remove bacteria.

EXAMPLE 2

Two test samples of stroma - free 0.04 g% hemoglobin solutions in 0.1 M sodium phosphate buffer with 92 mM sodium dithionite were heated in sealed vials without head space in a water bath for 10 hours at 56°C. Controls were: 1) otherwise identical hemoglobin solution except for the absence of sodium dithionite and 2) the dithionite-containing solution which was not heated but stored at room temperature for 10 hours. One of the test samples was dialyzed to remove the dithionite. Aliquots were withdrawn from both test samples and both controls and the O.D. determined over the range of 400 to 700 millimicrons. The results are shown in Fig. 1. The test sample and unheated control containing the reducing agent both showed the typical deoxyhemoglobin spectrum. The test sample from which dithionite had been removed exhibits the oxyhemoglobin spectrum, which is biologically active. On the other hand, the heated control hemoglobin is by spectral analysis virtually all methemoglobin, biologically inactive. Thus, the biological activity of the hemoglobin is retained in the presence of reducing agent but is lost upon heating without reducing agent.

EXAMPLE 3

The procedure of Example 1 is repeated in this contemplated example with the heated test composition containing reductant. This composition was divided into 3 aliquots which respectively were seeded with sindbis, encephalomyocarditis (EMC), and adeno type 5 virus so that the concentration of virus was, respectively, 6-7 $\log_{10}$ plaque forming units (PFU)/ml, 4 $\log_{10}$ PFU/ml and 4.5 $\log_{10}$ tissue culture 50% infective dose (TCID - 50)/ml.

The TCID designation may be explained as follows: In biological quantitation, the end point is usually taken as the dilution at which a certain proportion of the test system cells react or die. The 100% end point is frequently used. However, its accuracy is greatly affected by small chance variations. A desirable end point is one representing a situation in which one-half of the test system reacts while the other one - half does not. The best method is to use large numbers of test systems at closely spaced dilutions near the value for 50% reaction and then interpolate a correct value. The negative logarithm of the

$$TCID_{50} \text{ end point titer} =$$

$$\begin{pmatrix} \text{Negative logarithm of} \\ \text{the highest virus con-} \\ \text{centration used} \end{pmatrix} - \left[ \left( \frac{\text{Sum of \% mortality}}{\text{at each dilution}} \middle/ 100 - 0.5 \right) \times \begin{pmatrix} \text{Logarithm} \\ \text{of} \\ \text{Dilution} \end{pmatrix} \right]$$

The tissue culture 50% end point represents a viral titer that gives rise to cytopathic changes in 50% of the cells in an inoculated culture. In applying the above technique for determination of concentration, logarithmic dilutions are prepared in minimum essential medium plus 2% fetal calf serum. 0.2 ml of each dilution is added to replicate cultures of BGMK (Buffalo Green Monkey Kidney) cells in microtiter plates. The inoculated cultures are incubated at 36°C under 5% $CO_2$ and observed microscopically over a period of 7 to 8 days. The percent mortality of cells in a culture at a given dilution is determined by observing for cellular degeneration, as evidenced by refractile cells. The TCID-50 can then be calculated as shown above.

The EMC and sindbis virus infective titer is obtained by preparing dilutions of viral suspension as described above. BGMK cell monolayers were prepared in 35mm petri dishes. Viral adsorption to the cells was initiated by adding 0.2 ml of suspension to the monolayer. After 1 hour, the monolayer was overlaid with 2 ml of nutrient agar medium and incubated for 24-72 hours at 37°C. The plaques which formed were then made visible by staining the cells with neutral red at 1:2000 by weight in saline.

The results with virus were subjected to regression analysis with the method of least squares to allow the fitting of a linear line to the data and plotted. Similar results were obtained with all viruses. The viral infective titer in all three aliquots was reduced significantly by the method 1 heat tratment, thereby reducing the risk of patient infection by hepatitis or other viruses.

EXAMPLE 4

The method of Example 1 was repeated in this contemplated example except that the stroma-free hemoglobin had been crosslinked by 3,5-dibromosalicyl-bis- fumarate and subsquently pyridoxalated in accord with the method disclosed by Tye et al., in Bolin et al., editors, Advances in Blood Substitute Research, New York, Alan R. Liss, (1983) and literature cited therein.

**Claims**

1. A method for reducing the viral biological infectivity of a hemoglobin composition suspected to contain such infectivity, comprising heating the hemoglobin composition and a reducing agent under conditions such that the virus is rendered substantially biologically inactive but the hemoglobin is not substantially biologically inactivated, the reducing agent acting as herein defined to maintain the hemoglobin in the deoxy form during heating, and processing the solution for therapeutic use.

2. The method of Claim 1 wherein the condition is the time of heating.

3. The method of Claim 2 wherein the time of heating is from 5 to 15 hours for aqueous solutions of the composition and from 20 to 96 hours for dry compositions.

4. The method of Claim 1, 2 or 3 wherein the heating temperature is from 50°C to 80°C.

5. The method of any preceding claim wherein the reducing agent is a reduced dye, sulfoxy or sulfhydryl compound.

6. The method of Claim 5 wherein the agent is dithionite, bisulfite, metabisulfite, sulfite, reduced glutathione or dithiothreitol.

7. The method of Claim 1, 2, 3 or 4 in which the reducing agent has a reducing potential which is greater than ascorbate.

8. The method of any preceding claim wherein the composition is crosslinked and/or pyridoxalated.

9. A therapeutic heat treated stroma-free composition for administration to a patient comprising biologically active hemoglobin for performing its oxygen transport function *in vivo*, which hemoglobin has been retained during heating in its deoxy form by a reducing agent as herein defined, biologically noninfectious virus and substantially no inactive hemoglobin.

10. The composition of Claim 9 wherein the biologically inactive hemoglobin is methemoglobin.

11. The composition of Claim 9 or 10 wherein the biologically noninfectious virus is immunologically detectable but is substantially biologically noninfective.

12. The composition of Claim 9, 10 or 11 containing from 1 to 15 mole % biologically inactive hemoglobin.

13. The composition of Claim 9, 10, 11 or 12, wherein the hemoglobin is crosslinked and/or pyridoxalated.

14. The composition of Claim 9, 10, 11, 12 or 13 further comprising a reducing agent.

15. An aqueous solution of stroma-free and biologically active hemoglobin in accordance with Claim 9, substantially free of the risk of contamination by active virus (reduction of at least 3 logs of viral activity), having an oxygen affinity of about from 24 to 32 mm Hg.


**Patentansprüche**

1. Verfahren zum Verringern der viralen biologischen Infektiosität einer Hämoglobinzusammensetzung, die unter dem Verdacht steht, eine solche Infektiosität zu enthalten, wobei das Verfahren folgende Schritte aufweist: Erwärmen der Hämoglobinzusammensetzung und eines Reduktionsmittels unter solchen Bedingungen, daß das Virus im wesentlichen biologisch inaktiv gemacht wird, aber das Hämoglobin nicht wesentlich biologisch inaktiviert wird, wobei das Reduktionsmittel der hier definierten Art so wirkt, daß es das Hämoglobin während des Erwärmens in der Desoxy-Form hält, und Verarbeiten der Lösung für therapeutische Zwecke.

2. Verfahren nach Anspruch 1,
   wobei die Bedingung die Erwärmungsdauer ist.

3. Verfahren nach Anspruch 2,
   wobei die Erwärmungsdauer 5 bis 15 Stunden für wäßrige Lösungen der Zusammensetzung und 20 bis 96 Stunden für trockene Zusammensetzungen beträgt.

4. Verfahren nach Anspruch 1, 2 oder 3,
   wobei die Erwärmungstemperatur zwischen 50 °C und 80 °C liegt.

5. Verfahren nach einem der vorhergehenden Ansprüche, wobei das Reduktionsmittel eine reduzierte Farbstoff-, Sulfoxy- oder Sulfhydrylverbindung ist.

6. Verfahren nach Anspruch 5,
   wobei das Mittel Dithionit, Hydrogensulfit, Metabisulfit, Sulfit, reduziertes Glutathion oder Dithiothreitol ist.

7. Verfahren nach Anspruch 1, 2, 3 oder 4,
   wobei das Reduktionsmittel ein Reduktionspotential hat, das größer als das von Ascorbat ist.

8. Verfahren nach einem der vorhergehenden Ansprüche, wobei die Zusammensetzung vernetzt und/oder pyridoxaliert ist.

9. Wärmebehandelte therapeutische stromafreie Zusammensetzung zur Verabreichung an einen Patienten, die folgendes aufweist: biologisch aktives Hämoglobin zur Durchführung seiner Sauerstofftransportfunktion in vivo, wobei das Hämoglobin während des Erwärmens durch ein Reduktionsmittel der hier definierten Art in seiner Desoxy-Form gehalten worden ist, biologisch nichtinfektiöses Virus und im wesentlichen kein inaktives Hämoglobin.

**10.** Zusammensetzung nach Anspruch 9,
wobei das biologisch inaktive Hämoglobin Methämoglobin ist.

**11.** Zusammensetzung nach Anspruch 9 oder 10,
wobei das biologisch nichtinfektiöse Virus immunologisch nachweisbar, aber im wesentlichen biologisch nichtinfektiös ist.

**12.** Zusammensetzung nach Anspruch 9, 10 oder 11,
die 1 bis 15 Mol biologisch inaktives Hämoglobin enthält.

**13.** Zusammensetzung nach Anspruch 9, 10, 11 oder 12,
wobei das Hämoglobin vernetzt und/oder pyridoxaliert ist.

**14.** Zusammensetzung nach Anspruch 9, 10, 11, 12 oder 13,
die ferner ein Reduktionsmittel aufweist.

**15.** Wäßrige Lösung von stromafreiem und biologisch aktivem Hämoglobin gemäß Anspruch 9, die im wesentlichen frei von dem Risiko einer Kontaminierung durch aktives Virus ist (Reduktion auf wenigstens 3 log Virusaktivität) und eine Sauerstoffaffinität von etwa 24 bis 32 mmHg hat.

## Revendications

**1.** Procédé pour réduire l'infectivité biologique virale d'une préparation d'hémoglobine suspectée de contenir une telle infectivité, comportant le chauffage de la préparation d'hémoglobine et un agent réducteur dans des conditions telles que le virus soit essentiellement rendu biologiquement inactif mais que l'hémoglobine soit essentiellement non biologiquement inactivée, l'agent réducteur agissant comme ci-défini pour maintenir l'hémoglobine sous la forme déoxy durant le chauffage, et le traitement de la solution pour l'usage thérapeutique.

**2.** Procédé selon la revendication 1 dans lequel la condition est la durée de chauffage.

**3.** Procédé selon la revendication 2 dans lequel le temps de chauffage est compris entre 5 et 15 heures pour les solutions aqueuses de la préparation et entre 20 et 96 heures pour les préparations sèches.

**4.** Procédé selon la revendication 2 ou 3 dans lequel la température de chauffage est comprise entre 50°C et 80°C.

**5.** Procédé selon l'une quelconque des revendications précédentes dans lequel l'agent réducteur est un colorant réduit, un composant sulfoxy ou sulfhydryle.

**6.** Procédé selon la revendication 5 dans lequel l'agent est le dithionite, le bisulfite, le métabisulfite, le sulfite, la glutathione réduite ou le dithiothréitol.

**7.** Procédé selon l'une des revendications 1, 2, 3 ou 4 dans lequel l'agent réducteur a un pouvoir réducteur plus important que l'ascorbate.

**8.** Procédé selon l'une quelconque des revendications précédentes dans lequel la préparation est réticulée et/ou pyridoxalée.

**9.** Composition thérapeutique, traitée thermiquement et exempte de stroma pour administration à un patient, comprenant de l'hémoglobine biologiquement active pour réaliser la fonction de transport d'oxygène *in vivo*, ladite hémoglobine ayant retenu durant le chauffage sous sa forme deoxy au moyen d'un agent réducteur comme ci-défini, un virus biologiquement non infectieux et essentiellement de l'hémoglobine non active.

**10.** Composition selon la revendication 9 dans laquelle l'hémoglobine biologiquement inactive est la méthémoglobine.

**11.** Composition selon la revendication 8 ou 9 dans laquelle le virus biologiquement non infectieux est décelable immunologiquement mais est essentiellement non biologiquement infectieux.

12. Composition selon la revendication 9, 10 ou 11 contenant 1 à 15 mole % d'hémoglobine biologiquement inactive.

13. Composition selon la revendication 9, 10, 11 ou 12 dans laquelle l'hémoglobine est réticulée et/ou pyridoxalée.

14. Composition selon la revendication 9, 10, 11, 12 ou 13 comportant en outre un agent réducteur.

15. Solution aqueuse d'hémoglobine biologiquement active, exempte de stroma, selon la revendication 9, essentiellement dépourvue du risque de contamination par des virus actifs (réduction d'au moins 3 logs de l'activité virale), présentant une affinité pour l'oxygène d'environ 24 à 32 mmHg.

FIG. 1

WITH REDUCING AGENT, BEFORE HEATING

WITH REDUCING AGENT, AFTER HEATING

WITH REDUCING AGENT, AND HEATING FOLLOWED BY DIALYSIS

NO REDUCING AGENT, AFTER HEATING

OPTICAL DENSITY

WAVELENGTH ( MILLIMICRONS )